# EUROPÄISCHE PATENTANMELDUNG

(11) **EP 0 522 287 A2**
(43) Veröffentlichungstag der Anmeldung: **13.01.1993**
(21) Anmeldenummer: 92109409.0
(22) Anmeldetag: 03.06.1992
(51) Int. Cl.: C07C 67/58, C07C 69/30

(54) **Verfahren zur Entfernung von Glycerin aus Glyceriden**

(30) Priorität: 05.06.1991 DE 4118487
(71) Anmelder: Peter, Siegfried, Prof. Dr., D-91080 Uttenreuth-Weiher (DE)
(72) Erfinder: Peter, Siegfried, Prof. Dr., W-8525 Uttenreuth-Weiher (DE); Czech, Bernd, Dr. Dipl.-Ing., W-8520 Erlangen (DE); Weidner, Eckard, Dr. Dipl.-Ing., W-8520 Erlangen (DE)
(74) Vertreter: Weinhold, Peter, Dr.

(57) **Zusammenfassung**

Beschrieben wird ein Verfahren zur Entfernung von Glycerin und/oder Diglycerin aus Glyceriden und Glyceridgemischen durch Extraktion, das dadurch gekennzeichnet ist, daß man das Glycerid bzw. Glyceridgemisch in vorzugsweise Carbonyl- und Hydroxylgruppen-haltigen organischen Verbindungen, gegebenenfalls in Kombination mit Kohlenwasserstoffen, löst und aus der resultierenden Lösung das Glycerin mit Hilfe von Wasser herausextrahiert. Dabei können Glycerinkonzentrationen im zu reinigenden Material von 0,1 Gew.-% und darunter einfach erreicht werden.

## Beschreibung

Die vorliegende Erfindung betrifft ein Verfahren zur Entfernung von Glycerin bzw. Diglycerin aus Glyceriden, die damit verunreinigt sind, insbesondere ein Verfahren, bei dem der Glyceringehalt der Glyceride auf 0,1 Gew.-% und darunter, vorzugsweise sogar auf 0,01 Gew.-% und darunter verringert werden kann.

Mono- und Diglyceride haben emulgierende, stabilisierende, plastifizierende und verdickende Eigenschaften. Da die Mono- und Diester des Glycerins mit Fettsäuren eßbar sind, werden sie in verschiedenen Zweigen der Nahrungsmittelindustrie, der Pharmazie und Kosmetik eingesetzt. Die als Glycerinmonostearat, Glycerinmonooleat, Glycerinmonopalmitat usw. bezeichneten Gemische sind Wasser-in-Öl-Emulgatoren (W/O-Emulgatoren). Durch kleine Mengen an Zusätzen (Seifen, Polyethylenoxid-Verbindungen, sulfatierte Alkohole) werden sie selbstemulgierend und sind dann gute Öl-in-Wasser-Emulgatoren (O/W-Emulgatoren). In Abhängigkeit von der Art des Zusatzes können sie auch säurestabile und (in gewissen Grenzen) Elektrolyt-stabile Emulsionen liefern.

Die Monoglyceride höherer Fettsäuren (z.B. Glycerinmonostearat) können auch als Gleitmittel bei der Kunststoffverarbeitung eingesetzt werden. Durch Molekulardestillation gewonnene Monoglyceride mit einem Monoestergehalt von über 90% werden hauptsächlich in der Nahrungsmittelindustrie (Teig-, Süß- und Backhilfswaren, Margarine, Speiseeis) verwertet. Durch Umsetzung von Monoglyceriden mit Essigsäure erhält man flüssige bis wachsartige Monoglyceride (Acetoglyceride), die aufgrund ihrer Verträglichkeit mit pharmazeutischen Wirkstoffen und ihrer physiologischen Unbedenklichkeit in Lebensmitteln (Schutzfilm), Kosmetika und Pharmazeutika eingesetzt werden.

Durch Zusatz von Monoglycerid (bis zu 5% Palmitin/Stearinsäure und 90 bis 10% Palmitin-Stearinsäuremono/diglycerid) erreicht man selbstemulgierende Eigenschaften der zu Backzwecken bestimmten Backfette (als "superglycerolated shortenings" bezeichnet).

Aufgrund ihrer besonderen Struktur vermögen die Monoglyceride die plastifizierende wirkung von Stärke und Gluten bei der Teigbereitung zu verändern, indem sie sich in feiner Verteilung in die homogenen Plastifikate einschieben, sie unterbrechen und damit den Teig geschmeidiger machen, d.h. verkürzen. Gleichzeitig wird die Einarbeitung von Luft erleichtert, so daß zusammengenommen Backwaren mit vergrößerten Volumen und besserer "Kürze" anfallen.

Die Mono- und Diglyceride sind insbesondere durch Verestern von Glycerin mit Fettsäuren erhältlich. Ein anderer Weg ist die Umsetzung von Triglyceriden mit Glycerin. Weiterhin wird die enzymatische Zerlegung von Triglyceriden in neuerer Zeit intensiv untersucht. Bei der Veresterung, die gewöhnlich bei höherer Temperatur i.a. in Gegenwart eines Katalysators (z.B. einer Calcium- oder Natriumseife) erfolgt, stellt sich ein Gleichgewichtsgemisch von etwa 60% Mono-, 35% Di- und 5% Triglyceriden ein. Durch Variation der Reaktionsbedingungen ist es möglich, die Ausbeute an Monoglyceriden in weiten Grenzen zu variieren.

Nach Abkühlen des Reaktionsgemisches wird überschüssiges Glycerid als Unterphase durch Dekantieren abgetrennt. In der das Glyceridgemisch enthaltenden Oberphase sind in der Regel neben etwa 6 bis 20 Gew.-% freiem Glycerin noch 1 bis 2 Gew.-% freie Fettsäuren gelöst. Das Glycerin (und gegebenenfalls Diglycerin), welches bei der Umsetzung entstanden ist, wird vor dem Verkauf der Produkte entfernt. Bei den Produkten, die durch Molekulardestillation an Monoglyceriden angereichert wurden, kann die Entfernung des Glycerins auch nach der Destillation erfolgen. Die Entfernung des Glycerins geschieht durch Destillation und/ oder Wasserwäsche. Häufig wird freies Glycerin auch durch Dampfstrippen bei vermindertem Druck entfernt. In den Fällen, in denen Glycerin abdestilliert oder gestrippt wird, bleibt der Katalysator im Glyceridgemisch zurück. Durch die bekannten Verfahren gelingt es, den Glyceringehalt auf 1 bis 2 Gew.-% herabzusetzen. Ferner wurde vorgeschlagen, das Glycerin mit einer 5%-igen wäßrigen NaCl-Lösung auszuwaschen (Bailey's Industrial Oil and Fat Products, Band 2, 4. Auflage, John Wiley & Sons, New York 1982, Seite 139). Das Verfahren der wasserwäsche ist schwierig und im ganzen genommen nicht befriedigend wegen der hohen Grenzflächenaktivität des Materials und der Leichtigkeit, mit der Emulsionen gebildet werden. In Shortenings des Emulgator-Typs sind als Zusatz (6% Mono- und Diglyceride) nur Glyceride mit einem maximalen Gehalt von 0,30 Gew.-% an freiem Glycerin erlaubt.

Für die Verwendung im Nahrungsmittelbereich muß im allgemeinen der Katalysator aus dem Glyceridgemisch abgetrennt werden. Dies kann durch Adsorption oder durch Ionenaustausch geschehen. Das Glyceridgemisch wird dann durch Molekulardestillation weiter aufgetrennt. Es werden so Produkte mit einem Monoglyceridgehalt von 90 bis 95 Gew.-% erhalten. Die Anreicherung von Monoglyceriden aus laurinsäurereichen Produkten, wie beispielsweise Kokosöl oder Palmkernöl, ist auf dem Weg der Destillation dadurch erschwert, daß die Diglyceride kurzkettiger Fettsäuren (z.B. Capron-, Capryl- und Caprinsäure) gleichzeitig mit den Monoglyceriden der längerkettigen Fettsäuren verdampfen.

Bei den hohen Temperaturen im Filmverdampfer erfolgt in geringem Maße Disproportionierung, wobei freie Fettsäuren und Glycerin entstehen. Etwa 40% der Beschickung werden als hochprozentiges Monoglycerid erhalten. Das Glycerin reichert sich bei der Molekulardestillation im Destillat an. Infolgedessen sind nach der Molekulardestillation Maßnahmen zur Reduzierung des Gehalts an Glycerin und Diglycerin, das bei den hohen Temperaturen im Filmverdampfer (in der Regel etwa 200 bis 220°C) entsteht, erforderlich.

Neben den Mono- und Diglyceriden der C₁₈-C₁₆-Fettsäuren, die in der Nahrungsmittelindustrie, der Kosmetik, der Pharmazie und für technische Zwecke bevorzugt verwendet werden, haben in zunehmenden Umfang Glyceride von Fettsäuren geringerer Kettenlänge als Ausgangsprodukte für die Herstellung biologisch abbaubarer Tenside an Bedeutung gewonnen. Man geht dabei von Palmkern- und Kokosnußölen aus. In der nachfolgenden Tabelle ist das Fettsäuremuster einiger Pflanzenöle zusammengestellt. Die Angaben sind auf Prozent der Gesamtfettsäuren bezogen.

| Fettsäure | Sonnenblumenöl | Palmöl | Soyaöl | Kokosöl | Palmkernöl |
|---|---|---|---|---|---|
| Capronsäure | - | - | - | bis 0,8 | bis 0,2 |
| Caprylsäure | - | - | - | 7,8- 9,5 | 2,7- 4,3 |
| Caprinsäure | - | - | - | 4,5- 9,7 | 3,0- 7,0 |
| Laurinsäure | - | - | - | 44-51 | 47-52 |
| Myristinsäure | - | 0,6-2,4 | bis 0,4 | 13-18,5 | 14-17,5 |
| Palmitinsäure | 3,3-6,5 | 32-45 | 2,3-10,6 | 7,5-10,5 | 6,5- 8,8 |
| Stearinsäure | 1,3-3 | 4-6,3 | 2,4-6 | 1-3 | 1- 2,5 |
| Ölsäure | 14-43 | 38-53 | 23,5-31 | 5- 8,2 | 10,5-18,5 |
| Linolsäure | 44-68 | 6-12 | 49-51,5 | 1- 2,6 | 0,7- 1,3 |

Die Entfernung des Glycerins aus den Monoglyceriden der an Laurinsäure reichen Fette (auch "laurics" genannt) ist problematischer als die Entfernung des Glycerins aus den Monoglyceriden der Stearinsäure und Ölsäure, weil bei der Destillation mit abnehmendem Glyceringehalt zunehmend die Monoglyceride der kurzkettigen Säuren übergehen und so das Fettsäuremuster verändert wird.

Der vorliegenden Erfindung lag somit die Aufgabe zugrunde, ein Verfahren zur Entfernung von Glycerin und gegebenenfalls Diglycerin aus damit verunreinigten Glyceriden und Glyceridgemischen durch Extraktion bereitzustellen, das es insbesondere ermöglicht, den (Di)glyceringehalt auf Werte von 0,1 Gew.-% und darunter, insbesondere 0,1 bis 0,01 Gew.-%, zu verringern, bei dem das Problem der Emulsionsbildung bei der Extraktion nicht oder nur in geringem Maße auftritt, und das kontinuierlich durchgeführt werden kann.

Demgemäß ist Gegenstand der Erfindung ein Verfahren zur Entfernung von Glycerin und/oder Diglycerin aus Glyceriden und Glyceridgemischen durch Extraktion, das dadurch gekennzeichnet ist, daß man das Glycerid bzw. Glyceridgemisch löst in
(a) mindestens einer organischen Verbindung mit Carbonyl- und/oder Sulfoxid- und/oder Sulfongruppe, die eine Wasserlöslichkeit von höchstens 15 Gew.-%, vorzugsweise höchstens 10 Gew.-% (bei 20°C) und einen Siedepunkt von höchstens 200°C (bei Normaldruck) aufweist (Verbindungstyp A), entweder als solcher oder in Mischung mit bis zu 70 Gew.-%, vorzugsweise bis zu 50 Gew.-%, und insbesondere bis zu 10 Gew.-%, bezogen auf die Mischung, einer oder mehrerer Verbindungen, die ausgewählt sind aus Verbindungen des Typs A, mit der Ausnahme, daß die Wasserlöslichkeit mehr als 15 Gew.-%, vorzugsweise mehr als 20 Gew.-%, beträgt (Verbindungstyp B) und Hydroxylgruppenhaltigen Verbindungen mit einem Siedepunkt von höchstens 200°C (Verbindungstyp C); oder
(b) mindestens einer Verbindung des Typs A und/oder mindestens einer Verbindung des Typs B und/oder mindestens einer Verbindung des Typs C, wie oben definiert, in Mischung mit 5 bis 95, vorzugsweise 20 bis 80, und insbesondere 40 bis 60 Gew.-%, bezogen auf die Mischung, einer oder mehrerer Verbindungen aus der Gruppe der gegebenenfalls halogensubstituierten Kohlenwasserstoffe mit bis zu 20 Kohlenstoffatomen (Verbindungstyp D) und der Ether mit bis zu 20 Kohlenstoffatomen (Verbindungstyp E); oder
(c) mindestens einer Verbindung des Typs E, entweder als solcher oder in Mischung mit bis zu 80, vorzugsweise bis zu 70 und insbesondere bis zu 60 Gew.-%, bezogen auf die Mischung, mindestens einer Verbindung des Typs D; oder
(d) Kohlendioxid oder einer Mischung von Kohlendioxid mit bis zu 95, vorzugsweise 20 bis 80 und insbesondere 30 bis 70 Gew.-%, bezogen auf die Mischung an Propan und/oder Butan;
wobei die Menge an Lösungsmittel (a), (b), (c) oder (d) so gewählt wird, daß man eine 5 bis 80, vorzugsweise 10 bis 60 und insbesondere 20 bis 40 Gew.-%ige Lösung des zu reinigenden Glycerids bzw. Glyceridgemisches im Lösungsmittel erhält; und man aus der resultierenden Lösung das Glycerin (Diglycerin) mit Hilfe von Wasser herausextrahiert. (Dabei werden als Katalysatoren eingesetzte Seifen mit dem Glycerin extrahiert.)

Die nach dem obigen Verfahren zu reinigenden Glyceride bzw. Glyceridgemische weisen in der Regel einen Gesamtgehalt an Glycerin und Diglycerin von 0,2 bis 30, insbesondere 0,3 bis 25 und noch häufiger 1 bis 20 Gew.-% auf. Am häufigsten beträgt der Glyceringehalt 5 bis 17 Gew.-%.

Wie bereits oben erwähnt, handelt es sich bei den erfindungsgemäß von Glycerin bzw. Diglycerin zu befreienden Substanzen um Monoglyceride, Diglyceride oder Triglyceride bzw. irgendwelche Mischungen derselben. Dabei können selbstverständlich die Monoglyceride als solche wieder als Gemische von Monoestern des Glycerins mit verschiedenen Fettsäuren vorliegen. Entsprechendes gilt für die Diglyceride und Triglyceride. Besonders bevorzugt werden erfindungsgemäß Monoglyceride bzw. Diglyceride und Gemische derselben als Ausgangsmaterialien eingesetzt.

Bevorzugt werden die zu reinigenden Glyceridgemische durch Veresterung von Glycerin mit Carbonsäuren, Umesterung von Triglyceriden mit Glycerin oder enzymatische Spaltung von Triglyceriden oder Fraktionierung der so erhaltenen Gemische erhalten. Liegen Mischungen von Mono-, Di- und Triglyceriden vor, so weisen diese gewöhnlich die folgende Zusammensetzung auf, wobei die Prozentsätze auf die Gesamtmenge an Glyceriden und freien (Fett-)Säuren bezogen sind:
20 bis 99, vorzugsweise 30 bis 80 und insbesondere 40 bis 70 Gew.-% Monoglyceride;
0,4 bis 95, vorzugsweise 15 bis 60 und insbesondere 25 bis 45 Gew.-% Diglyceride;
0,3 bis 20, vorzugsweise 1 bis 15 und insbesondere 3 bis 10 Gew.-% Triglyceride;
0,3 bis 5, insbesondere 0,5 bis 3 Gew.-% freie, (Fett-)Säuren.

Die erfindungsgemäß zu reinigenden Glyceride und Glyceridgemische leiten sich in der Regel von (gegebenenfalls ungesättigten) aliphatischen Carbonsäuren (Fettsäuren) mit 4 bis 22, vorzugsweise 6 bis 18 Kohlenstoffatomen, ab. Beispiele für derartige Säuren sind Buttersäure, Capronsäure, Caprylsäure, Caprinsäure, Laurinsäure, Myristinsäure, Palmitinsäure, Stearinsäure, Ölsäure, Linolsäure, Linolensäure und Arachidonsäure.

Im folgenden soll auf die in den Lösungsmitteln (a), (b), (c) und (d) verwendeten Verbindungen näher eingegangen werden.

Verbindungen des Typs A mit einer Wasserlöslichkeit von höchstens 15 Gew.-% (und vorzugsweise höchstens 10 Gew.-%) sind z.B. Ester, Lactone, (vorzugsweise tertiäre) Amide, Lactame, Sulfoxide und Sulfone. Derartige Verbindungen können weitere funktionelle Gruppen tragen, vorausgesetzt, diese Gruppen können unter den Extraktionsbedingungen nicht mit den im zu reinigenden Ausgangsmaterial vorhandenen Verbindungen oder zumindest nicht mit den Glyceriden reagieren. Selbstverständlich müssen trotz Anwesenheit dieser funktionellen Gruppen die Bedingungen hinsichtlich Siedepunkt und Löslichkeit in Wasser erfüllt sein. Beispiele für geeignete funktionelle Gruppen, die zusätzlich vorhanden sein können, sind Hydroxy, Halogen (insbesondere F, Cl und Br) und NO₂. Es wird jedoch bevorzugt, Verbindungen des Typs A ohne zusätzliche funktionelle Gruppen einzusetzen. Selbstverständlich können die Verbindungen des Typs A auch mehrere Carbonyl-, Sulfoxid- oder Sulfongruppen im Molekül aufweisen. Beispiele hierfür sind Acetessigsäureester, Acetylaceton und Alkylester von mehrwertigen Carbonsäuren. Erfindungsgemäß bevorzugt als Verbindungen des Typs A werden Ester, Lactone und Ketone, insbesondere Ester und Lactone mit insgesamt 4 bis 8 Kohlenstoffatomen. Besondere Erwähnung verdienen hierbei Ethylacetat, Isopropylacetat und Butylacetat, von denen Ethylacetat nicht zuletzt auch wegen seiner physiologischen Unbedenklichkeit besonders bevorzugt wird.

Für die Verbindungen des Typs B gelten sinngemäß dieselben Ausführungen wie für die Verbindungen des Typs A, wobei jedoch zu berücksichtigen ist, daß die Verbindungen des Typs B eine Wasserlöslichkeit von mehr als, 15 Gew.-%, vorzugsweise mehr als 20 Gew.-% und insbesondere mehr als 50 Gew.-% aufweisen müssen. Besonders bevorzugt ist eine unbegrenzte Mischbarkeit mit Wasser. Typische Vertreter von Verbindungen mit Sulfon- oder Sulfoxidgruppen sind Dimethylsulfoxid und Sulfolan. Konkrete Beispiele für Amide sind Dimethylformamid und Dimethylacetamid. Bevorzugte Vertreter des Verbindungstyps B sind jedoch (cyclo-)aliphatische Ketone mit insgesamt 3 bis 6 Kohlenstoffatomen, wie z.B. Aceton, Butanon, 3-Pentanon und Cyclohexanon. Besonders bevorzugt werden hierbei Aceton und Butanon.

Die Verbindungen des Typs C weisen mindestens eine Hydroxylgruppe auf und sind vorzugsweise zu mindestens 20 Gew.-% in Wasser löslich. Ihr maximaler Siedepunkt beträgt wie bei den Verbindungen des Typs A und B 200°C, insbesondere maximal 160°C, vorzugsweise maximal 130°C. Verbindungen des Typs A, B und C mit einem Siedepunkt unter 100°C werden besonders bevorzugt.

Die Verbindungen des Typs C können neben der mindestens einen Hydroxylgruppe selbstverständlich weitere funktionelle Gruppen aufweisen, für deren Reaktivität bei den Extraktionsbedingungen jedoch dasselbe gilt, wie für die funktionellen Gruppen, die zusätzlich in den Verbindungen des Typs A und B vorhanden sein können. Zu den Verbindungen des Typs C zählen insbesondere (cyclo)-aliphatische Alkohole und Diole, wobei bei den Diolen eine der Hydroxylgruppen gegebenenfalls verethert sein kann (z.B. mit einer C₁-C₄-Alkylgruppe). Die bevorzugte Kohlenstoffzahl in den Verbindungen des Typs C beträgt 1 bis 6. Typische Vertreter dieser Verbindungsklasse sind Methanol, Ethanol, Isopropanol, n-Butanol und Ethylenglycol sowie Ethylenglycolmonomethyl- und Ethylenglycolmonoethylether. Besonders bevorzugt werden unter diesen Verbindungen Methanol, Ethanol, Isopropanol und n-Butanol, wobei für Ethanol (und in eingeschränktem Maße auch für Isopropanol) zusätzlich spricht, daß es für die Verwendung in Nahrungsmitteln zugelassen ist. Auch bei den Verbindungen des Typs C ist es besonders wünschenswert, wenn diese in jedem Verhältnis mit Wasser mischbar sind.

Die Verbindungen des Typs D sind gegebenenfalls halogensubstituierte Kohlenwasserstoffe mit bis zu 20 Kohlenstoffatomen. Unter den halogenierten Verbindungen sind insbesondere solche zu erwähnen, die Chloratome, gegebenenfalls in Kombination mit Fluoratomen, aufweisen. Typische Vertreter hierfür sind Methylenchlorid, Chloroform und Tetrachlorkohlenstoff sowie CHFCl₂ und CCl₂F₂. In der Regel wird es jedoch bevorzugt, halogenfreie Verbindungen des Typs D einzusetzen. Wenngleich auch aromatische Verbindungen, wie z.B. Toluol, eingesetzt werden können, sind die Verbindungen des Typs D vorzugsweise (cyclo)-aliphatischer Natur. Dabei weisen sie im allgemeinen 2 bis 12 Kohlenstoffatome und vorzugsweise 2 bis 7 Kohlenstoffatome auf. Bevorzugt werden unter diesen Verbindungen Propan und Butan, da diese für die Verwendung in Nahrungsmitteln zugelassen sind. Im Handel erhältliches Butan besteht überwiegend aus n-Butan, das ungefähr 5 bis 10 Gew.-% Isobutan enthält. Aufgrund der guten Zugänglichkeit kann für Nicht-Nahrungsmittelzwecke auch die Verwendung von im Handel erhältlichem Isohexan (Petrolether) angezeigt sein. Dieses Isohexan stellt ein Isomerengemisch dar, das im wesentlichen aus Kohlenwasserstoffen mit 5 bis 7 Kohlenstoffatomen besteht.

Wenngleich für den Einsatz im Lösungsmittel (b) Verbindungen des Typs D bevorzugt werden, können diese erfindungsgemäß auch ganz oder teilweise durch Verbindungen des Typs E ersetzt werden. Die Verbindungen des Typs E sind vorzugsweise (cyclo)-aliphatische Ether, die bis zu 20 Kohlenstoffatome, insbesondere 3 bis 12, und bevorzugt 4 bis 8 Kohlenstoffatome aufweisen. Diese Ether enthalten vorzugsweise 1 bis 3 Ethergruppen. Konkrete Beispiele hierfür sind Diethylether, Di(iso)propylether, Tetrahyrofuran, Dioxan, Trioxan, Ethylenglycoldialkylether und Diethylenglycoldialkylether (wobei die Alkylgruppen vorzugsweise 1 bis 4 Kohlenstoffatome aufweisen). Besonders bevorzugte Verbindungen des Typs E sind Diethylether, Di(iso)-propylether, Tetrahydrofuran und Dioxan.

Das erfindungsgemäße Verfahren wird vorzugsweise mit einer solchen Wassermenge durchgeführt, daß diese das 1- bis 8-fache, vorzugsweise das 2- bis 5-fache der Menge beträgt, die zur Erzeugung eines Zweiphasensystems (zumindest in der letzten Extraktionsstufe) erforderlich ist. Beim Extraktionswasser muß es sich selbstverständlich nicht notwendigerweise um reines Wasser handeln, sondern es kann (insbesondere anorganische) Zusätze wie z.B. Salze, darin gelöst enthalten, wenngleich dies in der Regel nicht bevorzugt ist. Lediglich bei der Reinigung eines Glyceridgemisches, das durch enzymatische Spaltung von Triglyceriden erhalten wurde, kann es vorteilhaft sein, die wäßrige Phase durch einen geeigneten Zusatz alkalisch zu machen, um dadurch neben dem Glycerin gleichzeitig auch die freien Fettsäuren aus dem Gemisch zu extrahieren.

Insbesondere wenn das erfindungsgemäße Verfahren kontinuierlich durchgeführt werden soll, was bevorzugt wird (insbesondere in Form einer Gegenstromextraktion), empfiehlt es sich, Extraktionswasser einzusetzen, das bereits die Gleichgewichtskonzentration (± 2, und vorzugsweise ± 1%) an den Verbindungen des Typs B und/oder C (und gegebenenfalls A) enthält, die sich im Verlauf der Extraktion in der wäßrigen Phase einstellen würde. Wird nach der Extraktion das Lösungsmittel zurückgewonnen und im Kreislauf geführt, stellt sich die Gleichgewichtskonzentration im Laufe der Zeit von selbst ein.

Prinzipiell kann das erfindungsgemäße Verfahren bei allen Temperatur- und Druckbedingungen durchgeführt werden. Besonders bevorzugt wird es jedoch, die Extraktion bei einer Temperatur von Raumtemperatur bis zum Schmelzpunkt (und gegebenenfalls bis zu 10°C über dem Schmelzpunkt) des Glyceridgemisches durchzuführen. Insbesondere wenn die Verfahrenstemperatur nahe dem Schmelzpunkt der Glyceride liegt, wird gewährleistet, daß sich diese im Laufe der Extraktion nicht abzuscheiden beginnen, was ansonsten insbesondere bei der kontinuierlichen Arbeitsweise zu Problemen führen kann. Andererseits muß jedoch berücksichtigt werden, daß Glyceride nicht unbegrenzt temperaturstabil sind, so daß Temperaturen, die wesentlich über der Schmelztemperatur der Glyceride liegen, normalerweise nicht empfehlenswert sind. Vorzugsweise beträgt die maximale Verfahrenstemperatur 50 bis 85°C, abhängig auch von der thermischen Stabilität der zu reinigenden Glyceride.

Prinzipiell kann das erfindungsgemäße Verfahren, abhängig vom Lösungsmittel, sowohl bei Normaldruck als auch Über- bzw. Unterdruck durchgeführt werden. Werden jedoch als Lösungsmittelkomponenten Verbindungen eingesetzt, die bei der Verfahrenstemperatur unter Normaldruck gasförmig sind, muß selbstverständlich unter Überdruck gearbeitet werden. Dies gilt insbesondere bei Verwendung des Lösungsmittelsystems (d), aber auch im Falle der Lösungsmittel (b) und (c), wenn z.B. gasförmige Kohlenwasserstoffe als Lösungsmittelkomponenten eingesetzt werden.

Da wie oben erwähnt mit dem erfindungsgemäßen Verfahren Glycerinkonzentrationen von 0,1 Gew.-% und darunter leicht erzielt werden können, sind weitere Maßnahmen zur Glycerinabtrennung, beispielsweise nach der Aufkonzentrierung von Monoglyceriden, nicht erforderlich. Weiter kann das erfindungsgemäße Verfahren bei niedrigen Temperaturen durchgeführt werden, bei denen eine Veränderung der Glyceride ausgeschlossen werden kann. Erfindungsgemäß können die Glyceride nach Durchführung des erfindungsgemäßen Verfahrens mühelos in Ausbeuten über 98% zurückgewonnen werden.

Überraschenderweise wurde beobachtet, daß wenn z.B. einer Lösung aus einem Glyceridgemisch in einem Kohlenwasserstoff, der eine größere Menge eines mit Wasser in jedem Verhältnis mischbaren Alkanols enthält, eine solche Wassermenge zugegeben wird, daß zwei coexistierende Phasen entstehen, die Unterphase das Glycerin und Diglycerin fast vollständig enthält, während die Oberphase das Glyceridgemisch enthält. Die Unterphase enthält nur geringe Mengen an Glyceriden. Der Verteilungsfaktor von Glycerin und Diglycerin zwischen Unterphase und Oberphase ist überraschend hoch (i.a. höher als 10). Die Konzentration an Glyceriden in der Unterphase ist gering, so daß nur geringe Verluste an Monoglyceriden bei der Extraktion des Glycerins entstehen. Die i.a. verwendeten Katalysatoren gehen dabei bevorzugt in die Unterphase. Als Katalysatoren werden üblicherweise (basische) Salze, wie z.B. Erdalkali- und Alkalimetallverbindungen, verwendet. Der Gehalt an Katalysatoren im Glycerid oder im Glyceridgemisch wird im Verlauf der Extraktion nach dem Verfahren dieser Erfindung erheblich vermindert. Meistens erübrigt sich so ein gesonderter Verfahrensschritt zur Katalysatorentfernung aus dem Endprodukt.

Monoglyceride sind in Kohlenwasserstoffen nur relativ wenig löslich. Durch Zusatz von z.B. Alkanolen, die im Wasser sehr gut löslich sind, wird die Löslichkeit der Glyceride in Kohlenwasserstoffen so weit erhöht, daß Konzentrationen von bis zu 80 Gew.-% ohne weiteres erzielt werden können. Die Extraktion des Glycerins aus einem Glyceridgemisch mit Wasser wird durch die große Neigung zur Emulsionsbildung sehr behindert. Die Phasentrennung verläuft in dem System sehr langsam. Das führt dazu, daß der Gehalt an Glycerin durch die Extraktion praktisch nur auf Werte von etwa 1% vermindert werden kann. Durch Destillation oder Dampfstrippen unter vermindertem Druck werden mit großem Aufwand Werte von 0,3 Gew.-% erreicht. Beispielsweise bewirkt nun die Zugabe eines in Wasser sehr gut löslichen Alkanols zu dem Kohlenwasserstoff überraschenderweise nicht nur eine Erhöhung der Löslichkeit der Monoglyceride im Kohlenwasserstoff, sondern auch eine Unterdrückung der Emulsionsbildung bei der Extraktion des Glycerins mit Wasser. Daraus resultiert eine bedeutende Beschleunigung der Phasentrennung nach intensiver Durchmischung im quasi-quaternären System aus Kohlenwasserstoff, Alkanol, Wasser und Glyceridgemisch. Obwohl sich das Alkanol in etwa zu gleichen Teilen in den coexistierenden Phasen verteilt, beobachtet man eine hohe Selektivität für die Extraktion des Glycerins durch die wäßrige Phase. Infolge der schnellen Phasentrennung kann eine wirtschaftlich vorteilhafte Gegenstromextraktion durchgeführt werden, die bei drei theoretischen Trennstufen bereits zu Glyceringehalten unter 0,1 Gew.-% führt, wenn von einer Anfangskonzentration von ca. 12 Gew.-% ausgegangen wird.

Das Entfernen des Glycerins aus Glyceridgemischen, die bei der Umesterung oder enzymatischen Lipolyse entstehen, mit Hilfe einer Wasserwäsche ist lange bekannt und wird teilweise in der Technik angewandt. Infolge von Emulsionsbildung wird das Verfahren jedoch unhandlich und eine vollständige Entfernung des Glycerins wird dadurch unmöglich. Beim erfindungsgemäßen Verfahren tritt überraschenderweise keinerlei Emulsionsbildung auf. Unterphase und Oberphase trennen sich ausreichend schnell um einen mehrstufigen Gegenstromprozeß zu realisieren. Es gelingt auf diese Weise, den Glyceringehalt der Glyceride bzw. Glyceridgemische auf Werte kleiner als 0,1 Gew.-% zu verringern.

In einem mehrstufigen Extraktionsprozeß kann im Gegenstrom mit Hilfe von Wasser, das z.B. die Gleichgewichtskonzentration an Alkanol enthält, aus der Lösung eines (di)glycerinhaltigen Glyceridgemisches in einem Kohlenwasserstoff, welcher ein Alkanol im entsprechenden Verhältnis zur Glyceridkonzentration enthält, (Di)glycerin praktisch vollständig entfernt werden.

Erwähnt werden sollte auch, daß das erfindungsgemäße Verfahren ohne Probleme sowohl absatzweise als auch kontinuierlich durchgeführt werden kann. Für diesen Zweck eignet sich z.B. die Extraktion in einer gepackten Säule im Gegenstrom, in einer Kolonne mit rotierenden Einbauten, in einer Mischer-Abscheider-Batterie, in einem Zentrifugalextraktor oder in einer Siebbodenkolonne. Mit Ausnahme der Verwendung eines Zentrifugalextraktors kann die Extraktionswirkung durch Pulsation noch weiter verbessert werden, wenngleich die Pulsation in der Regel nur bei kleinen Materialmengen bequem durchzuführen ist.

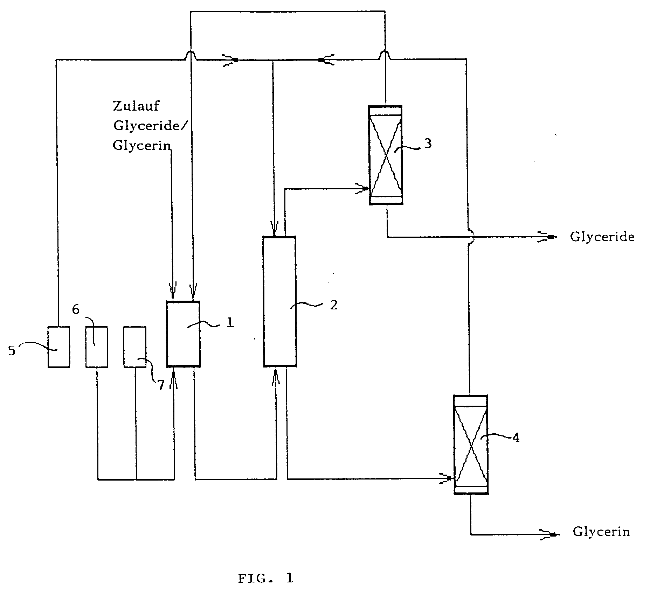

Das erfindungsgemäße Verfahren soll nun unter Bezugnahme auf die Figur 1 am Beispiel einer (kontinuierlichen) Glycerin-Extraktion unter Verwendung eines Alkanol/Kohlenwasserstoff-Lösungsmittelgemisches veranschaulicht werden.

Die Extraktion findet im Gegenstrom im Extraktor 2 statt. Dem Extraktor wird ein Gemisch aus Alkanol und Wasser als Extraktionsmittel für das Glycerin zugeführt. Das zu extrahierende (reinigende) Glyceridgemisch wird im Zulauftank und Mischer 1 in einem Alkanol/Kohlenwasserstoff-Gemisch zu einer klaren Lösung gelöst. Diese Lösung wird dem Extraktor 2 als Zulauf zugeführt und durchströmt denselben im Gegenstrom zum Extraktionsmittel. Die vom Glycerin befreite Lösung des Glyceridgemisches, das Raffinat, verläßt den Extraktor 2 und wird der Destillationskolonne 3 zugeführt.

In der Destillationskolonne 3 wird das Raffinat durch Rektifikation in das Lösungsmittel und das Glyceridgemisch zerlegt. Falls die Glyceride temperaturempfindlich sind, wird die Rektifikation unter vermindertem Druck durchgeführt. Das abgetrennte Lösungsmittel enthält neben Alkanol und Kohlenwasserstoff noch etwas Wasser, welches im Raffinat gelöst ist. Es wird dem Mischer 1 zugeführt. Das vom Glycerin befreite Glycerid oder Glyceridgemisch wird als Sumpfprodukt aus Kolonne 3 abgezogen.

Der Extrakt besteht aus einem Gemisch aus Wasser, Alkanol und Glycerin, das noch geringfügige Mengen an Glyceriden und gegebenenfalls freien Fettsäuren enthält und wird der Destillationskolonne 4 zugeführt. Durch Rektifikation werden Wasser und Alkanol als Kopfprodukt vom Glycerin getrennt, während das Sumpfprodukt aus Glycerin besteht, in welchem noch geringe Mengen Wasser und die in das Extrakt übergegangenen Glyceride gelöst sind. Das Kopfprodukt aus Wasser und Alkanol wird dem Extraktor 2 zugeführt, wobei etwaige Verluste an Wasser, Alkanol und Kohlenwasserstoff aus den Vorratsbehältern 5 (für H₂O), 6 (für Kohlenwasserstoffe) und 7 (für Alkanol) durch Zupumpen zum Mischer 1 ausgeglichen werden.

Das aufzubereitende Glyceridgemisch oder Glycerid wird dem Mischer 1 zweckmäßigerweise in flüssiger Form zugeführt. Seine Temperatur befindet sich somit oberhalb der betreffenden Schmelztemperatur. Nach Auflösung der Glyceride in Alkanol/Kohlenwasserstoff-Gemisch kann die Temperatur gegebenenfalls wieder erniedrigt werden und die Extraktion beispielsweise bei Raumtemperatur erfolgen. Wie oben erwähnt, bestehen hinsichtlich der Temperatur, bei der die eigentliche Extraktion erfolgt, grundsätzlich keine Einschränkungen. Normalerweise wird jedoch der Temperaturbereich von 20 bis 50°C für die Extraktion des Glycerins nach dem erfindungsgemäßen Verfahren bevorzugt.

Werden als Kohlenwasserstoff z.B. Propan oder Butan eingesetzt, muß die Extraktion unter Drücken erfolgen, die gleich oder etwas höher sind als der Dampfdruck von Propan bzw. Butan bei der jeweiligen Extraktionstemperatur. Beispielsweise beträgt bei 20°C der Dampfdruck von Propan 8,5 bar und der von Butan 2 bar.

Die folgenden Beispiele dienen zur weiteren Erläuterung des erfindungsgemäßen Verfahrens, ohne dieses zu beschränken. Soweit nicht anders angegeben, beziehen sich Prozentsätze auf das Gewicht.

### BEISPIEL 1

100 g eines Gemisches aus 64% Monoglyceriden, 30% Diglyceriden, 3,5% Triglyceriden, 1% freien Fettsäuren und 1,5% Glycerin wurden bei Raumtemperatur in einem Gemisch aus 70 g Ethanol und 70 g Isooctan (handelsübliches Isomerengemisch von Kohlenwasserstoffen mit im wesentlichen 7 bis 9 Kohlenstoffatomen) unter Bildung einer klaren Lösung aufgelöst. Dieser Lösung wurden sodann 150 g Wasser zugegeben. Nach intensivem Durchmischen bildeten sich innerhalb von 30 Minuten zwei Phasen, wobei die Oberphase 97 g des Glyceridgemisches und 0,3 g Glycerin und die Unterphase 3 g Glyceridgemisch und 1,2 g Glycerin enthielt. Daraus ergibt sich ein Trennfaktor von 129. Nach Eindampfen der Unterphase blieb ein Gemisch, das 60,2% Glyceride enthielt, zurück. Das Ethanol verteilte sich etwa gleichmäßig auf beide Phasen.

### BEISPIEL 2

100 g eines Gemisches aus 48,8 g Mono-, 30,3 g Di- und 5,1 g Triglyceriden sowie 15,8 g Glycerin wurden in einem Gemisch aus 70 g Ethanol und 70 g Isohexan (handelsübliches Isomerengemisch von Kohlenwasserstoffen mit im wesentlichen 5 bis 7 Kohlenstoffatomen) unter Bildung einer klaren Lösung aufgelöst. Diese Lösung wurde mit 60 g Wasser kräftig durchmischt. Nach dem Absetzen entstanden zwei Phasen, wobei die Oberphase 47,2 g Mono-, 29,3 g Di- und 4,9 g Triglyceride sowie 1,8 g Glycerin enthielt. In einer zweiten Extraktionsstufe wurde die Oberphase aus 81,4 g Glyceriden, 1,8 g Glycerin, 70 g Isohexan, 8 g Ethanol und 9 g Wasser mit einem Gemisch aus 60 g Wasser und 50 g Ethanol versetzt und durchmischt. Nach Absetzen der beiden Phasen wurde eine Oberphase erhalten, die sich aus 78,6 g Glyceriden, 0,09 g freiem Glycerin, 70 g Isohexan, 7 g Ethanol und 8 g Wasser zusammensetzte.

In einer dritten Extraktionsstufe wurde die Oberphase aus der zweiten Stufe wiederum mit einem Gemisch aus 60 g Wasser und 50 g Ethanol durchsetzt. In der Oberphase, die sich nach kräftigem Schütteln und anschließendem Absetzen bildete, konnte selbst mit Hilfe der Kapillar-Gaschromatographie kein Glycerin mehr nachgewiesen werden.

### BEISPIEL 3

100 g eines Gemisches aus 58 g Monoglyceriden, 35 g Diglyceriden, 5,5 g Triglyceriden, 1 g freien Fettsäuren und 0,44 g Glycerin wurden in einem Gemisch aus 100 g Isohexan und 50 g Ethanol gelöst. Der Lösung wurden sodann 75 g Wasser zugesetzt und das resultierende System wurde gut miteinander vermischt. In der Ruhe bildeten sich zwei Phasen, von denen die Oberphase 98% der eingesetzten Glyceride und 24% des eingebrachten Glycerins enthielt. 2% der eingesetzen Glyceride und 76% des Glycerins befanden sich in der Unterphase. Das Volumenverhältnis von Oberphase zu Unterphase betrug etwa 3,4:1.

### BEISPIEL 4

100 g eines Gemisches aus 58 g Monoglyceriden, 36 g Diglyceriden, 5,5 g Triglyceriden, 1 g freien Fettsäuren und 0,44 g Glycerin wurden in 200 g Methanol und 200 g Isohexan gelöst. Die Lösung wurde anschließend mit 150 g Wasser gemischt und gerührt. Nach Unterbrechung des Rührens bildeten sich etwa zwei gleichgroße flüssige Phasen. Die Oberphase enthielt 98 g des zugegebenen Gemisches. Glycerin konnte in der Oberphase nicht mehr nachgewiesen werden, d.h., das gesamte Glycerin war in die Unterphase übergegangen.

### BEISPIEL 5

157 g eines Monostearats (0,35% Glycerin, 0,36% Diglycerin, 1,03% freie Fettsäuren, 92,48% Monoglyceride, 0,32% Diglycerin-Monoglycerid, 5,25% Diglyceride, 0,21% Triglyceride) wurden bei 60°C und 6,5 bar mit 303 g Ethanol, 333 g Butan und 362 g Wasser gemischt. Nach Unterbrechung des Mischvorgangs bildeten sich zwei flüssige Phasen. Die in der Oberphase gelösten Inhaltsstoffe hatten nach Abzug der Lösungsmittel folgende Zusammensetzung: 0,10% Glycerin, 0,59% freie Fettsäuren, 99,31% Glyceride. Diglycerin war nicht nachweisbar. Die Inhaltsstoffe der Unterphase nach Abzug der Lösungsmittel bestanden aus 18,46% Glycerin, 19,01% Diglycerin, 1,2% freien Fettsäuren und 62,53% Glyceriden. Triglyceride waren in der Unterphase nicht nachweisbar.

### BEISPIEL 6

50 g eines Gemisches (aus Kokosöl), das aus 56% Monoglyceriden, 40% Diglyceriden, 3,5% freien Fettsäuren und 0,44% Glycerin bestand, wurden in einem Gemisch aus 125 g Isohexan und 125 g Isopropanol gelöst und anschließend mit 70 g Wasser vermischt. Nach Unterbrechung des Mischvorgangs bildeten sich zwei Phasen, von denen die Oberphase 16,0% und die Unterphase 0,81% an schwerflüchtigen Bestandteilen enthielt. Bei einem Phasen-Volumenverhältnis von etwa 5:1 waren 98% der eingesetzten Glyceride in der Oberphase gelöst. Der Glyceringehalt der in der Oberphase gelösten Glyceride nach Abzug der Lösungsmittel betrug 0,13%. In die Unterphase waren 0,16 g Glycerin übergegangen (entsprechend einer Konzentration von 2,67%, bezogen auf das gelöste schwerflüchtige Material).

### BEISPIEL 7

94 g eines Gemisches aus 46% Monoglyceriden, 32% Diglyceriden, 3,5% Triglyceriden, 2% Diglycerin-Monoglyceriden, 1,2% freien Fettsäuren, 16,3% freiem Glycerin und 0,3% Diglycerin wurden mit 220 g Ethanol und 295 g Wasser in einen 1-Liter-Autoklaven gegeben. Der Autoklav wurde auf 110°C erwärmt und darauf wurde unter Rühren bei einem Druck von 80 bar Propan in den Autoklaven eingepumpt. Es entstanden zwei Phasen, wie die Beobachtung durch ein Sichtfenster ergab. Aus der Oberphase und aus der Unterphase wurden Proben entnommen, wobei durch Nachpumpen von Propan der Druck konstant gehalten wurde. Die Oberphase enthielt 74,5 g Glyceridgemisch und 0,75 g freies Glycerin. Diglycerin konnte in der Oberphase nicht nachgewiesen werden. Die schwerflüchtigen Anteile in der Oberphase enthielten somit 1,02% freies Glycerin. Das schwerflüchtige Material in der Unterphase bestand aus 15,8 g freiem Glycerin, 0,3 g Diglycerin und 2,4 g Glyceridgemisch. Bezogen auf das schwerflüchtige Material betrug der Gehalt an freiem Glycerin und Diglycerin in der Unterphase 87%.

### BEISPIEL 8

50 g eines Stearinsäureglyceridgemisches aus 58% Monoglyceriden, 36% Diglyceriden, 3,6% Triglyceriden, 1% freien Fettsäuren und 1,4% freiem Glycerin wurden in einem Gemisch aus 100 g Aceton und 100 g Isohexan gelöst und anschließend mit 75 g Wasser vermischt. Nach Unterbrechung des Rührvorgangs bildeten sich zwei Phasen, wobei die Oberphase 49 g schwerflüchtiges Material gelöst enthielt. Dies entspricht 98% der eingesetzen Stearinsäureglyceride. Freies Glycerin konnte in der Oberphase selbst mit Hilfe der Gaschromatographie nicht nachgewiesen werden. Die Unterphase enthielt 1 g schwerflüchtiges Material, entsprechend 2% des eingesetzten Glyceridgemisches. Der Gehalt der Unterphase an freiem Glycerin betrug 0,7 g oder 70%, bezogen auf den Gehalt der Unterphase an schwerflüchtigem Material. Das Volumenverhältnis von Oberphase zu Unterphase betrug etwa 1,6:1.

### BEISPIEL 9

60 g Stearinsäureglyceride (58% Monoglyceride, 36% Diglyceride, 3,6% Triglyceride, 1% freie Fettsäuren und 1,4% freies Glycerin) wurden in einem Gemisch aus 120 g Isohexan und 120 g Ethylacetat gelöst und anschließend mit 66 g Wasser vermischt. Nach Unterbrechung des Mischvorganges bildeten sich zwei Phasen. Die Oberphase enthielt 56,2 g an gelösten schwerflüchtigen Bestandteilen, entsprechend 94% des eingesetzten Produktes. Freies Glycerin war in der Oberphase selbst mit Hilfe der gaschromatographischen Analyse nicht nachweisbar. Die Unterphase enthielt 3,8 g an schwerflüchtigen Bestandteilen, entsprechend 6% des Ausgangsmaterials. Der Gehalt an freiem Glycerin in der Unterphase betrug 22%, bezogen auf das schwerflüchtige Material. Das Volumenverhältnis von Oberphase zu Unterphase betrug etwa 3,6:1.

### BEISPIEL 10

200 g eines aus Kokosöl gewonnenen Glyceridgemischs (13,8 Gew.-% freies Glycerin) wurden mit 400 g Ethylacetat und 200 g Wasser in einem Scheidetrichter intensiv durchmischt. Die wäßrige und die organische Phase wurden nach abgeschlossener Phasentrennung separat aufgearbeitet. Die leichtflüchtigen Lösungsmittel Ethylacetat und Wasser wurden bei 85°C unter Wasserstrahlvakuum abgedampft. Der Gehalt an schwerflüchtigem Material in den koexistierenden Phasen betrug in der Oberphase 29,5 Gew.-% und in der Unterphase 16,5 Gew.-%. Die jeweiligen Glyceringehalte, bezogen auf das schwerflüchtige Material, betrugen in der Oberphase 0,65 Gew.-% und in der Unterphase 75,2 Gew.-%. Dies entspricht einem Trennfaktor zwischen Glycerin und den Glyceriden von 0,00245. Die zusammen mit dem Glycerin extrahierte Glyceridmenge war dementsprechend sehr gering. Die Glyceridverluste betrugen unter 2% der eingesetzten Glyceridmenge.

### BEISPIEL 11

100 g Ölsäureglyceridgemisch (55% Monoglyceride, 32% Diglyceride, 3% Triglyceride, 1% freie Fettsäuren und 9% freies Glycerin) wurden in einem Hochdrucksichtzellenautoklaven bei 80°C mit 80 g CO₂ vermischt. Der Druck im Autoklaven betrug 12 MPa. Es bildete sich eine dünnflüssige, homogene Phase aus Glyceriden und CO₂ aus. Mit Hilfe einer Hochdruckpumpe wurden 25 g Wasser zudosiert und 10 min intensiv mit den CO₂-gesättigten Glyceriden durchmischt. Nach Beendigung des Mischvorgangs trennten sich die beiden Phasen sehr schnell. Aus der Oberphase und der Unterphase wurde jeweils eine Probe entnommen. Dabei wurde der Druck im Autoklaven durch Zupumpen von CO₂ konstant gehalten. Das in den Proben gelöste CO₂ wurde im Verlauf der Entspannung freigesetzt und mit Hilfe eines Volumenzählers gemessen. Das in den so gewonnenen Proben enthaltene Wasser wurde in einer Vigreuxkolonne bei 80°C im Wasserstrahlvakuum abdestilliert. Die Menge an Wasser wurde durch Differenzwägung bestimmt. Der Gehalt der Oberphase an schwerflüchtigen Komponenten betrug 55%. Die Unterphase enthielt 21% schwerflüchtige Komponenten. Die gaschromatographische Analyse des schwerflüchtigen Materials ergab, daß der Glyceringehalt der in der Oberphase angereicherten Glyceride auf 2,0 Gew.-% vermindert worden war. Der Glyceringehalt im schwerflüchtigen Material der Unterphase betrug 77 Gew.-%. Etwa 1,5% der eingesetzten Glyceride waren in der Unterphase gelöst. Der Vorteil dieses Extraktionssystems ist, daß nur nichtbrennbare Lösungsmittel verwendet werden.

### BEISPIEL 12

100 g eines durch Glycerinolyse erzeugten Gemisches aus 12,4% Glycerin und 87,6% Glyceriden (60% Monoglyceride, Rest Diglyceride, Triglyceride, freie Fettsäuren) wurden mit 200 g Dioxan und 200 g Isohexan im Schütteltrichter versetzt. Es bildete sich eine wasserklare, homogene Flüssigphase. Zur Extraktion des Glycerins wurden 200 g Wasser zugegeben. Der Inhalt des Schütteltrichters wurde 5 Minuten intensiv durchmischt. Die anschließende Absetzzeit zur Phasentrennung betrug wenige Minuten. Die wäßrige Unterphase und die organische Oberphase wurden analysiert. Dabei wurden zunächst die Lösungsmittel abgedampft. Der Gehalt an schwerflüchtigem Material in der Oberphase betrug 25,3%. In der Unterphase waren 5,5% schwerflüchtiges Material enthalten. Der Glyceringehalt im schwerflüchtigen Material der Oberphase wurde durch die Extraktion auf 0,58% vermindert. Der Glyceringehalt im schwerflüchtigen Material der Unterphase betrug 72,1%. Etwa 4% der Glyceride waren in der Unterphase gelöst.

### BEISPIEL 13

1000 g eines aus Palmkernöl erzeugten Glyceridgemisches mit einem Glyceringehalt von 10,8% wurde im Beschickungsgefäß einer dreistufigen Mischer-Abscheiderbatterie mit 2000 g Tetrahydrofuran und 2000 g Isohexan gemischt. Es entstand sehr schnell eine klare, dünnflüssige Lösung. Im Extraktionsmittelgefäß wurden insgesamt 2000 g H₂O vorgelegt. Die zu extrahierende Lösung und das Extraktionsmittel wurden dem Extraktor mit Hilfe von Kreiselpumpen zudosiert. Die beiden Phasen wurden im Gegenstrom geführt. Insgesamt wurden 2400 g glycerinreiche Raffinatlösung und 4600 g Extraktlösung erhalten. Die Lösungsmittel aus Extrakt und Raffinat werden abgedampft. Der Gehalt an schwerflüchtigem Material betrug in der Extraktlösung 19,2% und in der Raffinatlösung 5,0%. Die Glyceringehalte betrugen im Extrakt, bezogen auf die Glyceride, weniger als 0,1% und im Raffinat 90%. Die zusammen mit dem Glycerin extrahierten Glyceride machten 1,3% der eingesetzten Glyceride aus.

### BEISPIEL 14

100 g eines aus Kokosöl erzeugten Glyceridgemisches mit einem Gehalt von 12,7% Glycerin wurden in einem Scheidetrichter mit 300 g Diisopropylether gemischt. Nach Wasserzugabe (300 g) und intensiver Durchmischung bilden sich innerhalb weniger Minuten zwei Phasen aus. Die untere Phase war zunächst noch trüb. Nach einer Standzeit von einigen Stunden war die Trübung verschwunden. Die beiden Phasen wurden getrennt aufgearbeitet. Abdestillieren des Ethers und des Wassers ergab einen Gehalt an schwerflüchtigem Material in der Oberphase von 22% und in der Unterphase von 6,0%. Die Glycerinkonzentrationen im schwerflüchtigen Material betrugen 0,18% in der Oberphase und 71% in der Unterphase. Das Volumenverhältnis von Oberphase zu Unterphase betrug etwa 1,24:1.

### BEISPIEL 15

100 g Ölsäureglyceride (55% Monoglyceride, 35% Diglyceride, 3% Triglyceride, 2% freie Fettsäuren und 5% freies Glycerin) wurden in einem Hochdrucksichtzellenautoklaven bei 60°C mit 70 g einer Mischung aus 51 Gew.-% CO₂ und 49 Gew.-% C₃H₈ vermischt. Der Druck im Autoklaven betrug 60 bar. Es bildete sich eine dünnflüssige, homogene Phase aus Glyceriden, Propan und CO₂ aus. Mit Hilfe einer Hochdruckpumpe wurden 130 g Wasser zudosiert und 10 min intensiv mit den gasgesättigten Glyceriden durchmischt. Nach Beendigung des Mischvorgangs trennten sich die beiden Phasen sehr schnell. Aus der Oberphase und der Unterphase wurde jeweils eine Probe entnommen. Dabei wurde der Druck im Autoklaven durch Zupumpen der Gasmischung konstant gehalten. Das in den Proben gelöste Gas wurde im Verlauf der Entspannung freigesetzt und mit Hilfe eines Volumenzählers gemessen. Das in den so gewonnenen Proben enthaltene Wasser wurde in einer Vigreuxkolonne bei 80°C im Wasserstrahlvakuum abdestilliert. Die Menge an Wasser wurde durch Differenzwägung bestimmt. Der Gehalt der Oberphase an schwerflüchtigen Komponenten betrug 41%. Die Unterphase enthielt 3 Gew.-% schwerflüchtige Komponenten. Die gaschromatographische Analyse des schwerflüchtigen Materials ergab, daß der Glyceringehalt der in der Oberphase angereicherten Glyceride auf 0,8% vermindert wurde. Der Glyceringehalt im schwerflüchtigen Material der Unterphase betrug 93%. Etwa 0,3% der eingesetzten Glyceride waren in der Unterphase gelöst.

## Patentansprüche

1. Verfahren zur Entfernung von Glycerin und/oder Diglycerin aus Glyceriden und Glyceridgemischen durch Extraktion, dadurch gekennzeichnet, daß man das Glycerid bzw. Glyceridgemisch löst in
(a) mindestens einer organischen Verbindung mit Carbonyl- und/oder Sulfoxid- und/oder Sulfongruppe, die eine Wasserlöslichkeit von höchstens 15 Gew.-% und einen Siedepunkt von höchstens 200°C aufweist (Verbindungstyp A), entweder als solcher oder in Mischung mit bis zu 70 Gew.-%, bezogen auf die Mischung, einer oder mehrerer Verbindungen, die ausgewählt sind aus Verbindungen des Typs A, mit der Ausnahme, daß die Wasserlöslichkeit mehr als 15 Gew.-% beträgt (Verbindungstyp B), und Hydroxylgruppen-haltigen Verbindungen mit einem Siedepunkt von höchstens 200°C (Verbindungstyp C); oder
(b) mindestens einer Verbindung des Typs A und/oder mindestens einer Verbindung des Typs B und/oder mindestens einer Verbindung des Typs C, wie oben definiert, in Mischung mit 95 bis 5 Gew.-%, bezogen auf die Mischung, einer oder mehrerer Verbindungen aus der Gruppe der gegebenenfalls Halogen-substituierten Kohlenwasserstoffe mit bis zu 20 Kohlenstoffatomen (Verbindungstyp D) und der Ether mit bis zu 20 Kohlenstoffatomen (Verbindungstyp E); oder
(c) mindestens einer Verbindung des Typs E, entweder als solcher oder in Mischung mit bis zu 80 Gew.%, bezogen auf die Mischung, mindestens einer Verbindung des Typs D; oder
(d) Kohlendioxid oder einer Mischung von Kohlendioxid mit bis zu 95 Gew.-%, bezogen auf die Mischung, Propan und/oder Butan;
wobei die Menge an Lösungsmittel (a), (b), (c) oder (d) so gewählt wird, daß man eine 5 bis 80 gew.-%ige Lösung des zu reinigenden Glycerids bzw. Glyceridgemisches im Lösungsmittel erhält,
und man aus der resultierenden Lösung das Glycerin (Diglycerin) mit Hilfe von Wasser herausextrahiert.

2. Verfahren nach Anspruch 1, dadurch gekennzeichnet, daß das zu reinigende Glycerid bzw. Glyceridgemisch einen Gesamtgehalt an Glycerin und Diglycerin von 0,2 bis 30 Gew.-%, vorzugsweise 5 bis 17 Gew.-%, aufweist.

3. Verfahren nach irgendeinem der Ansprüche 1 und 2, dadurch gekennzeichnet, daß das zu reinigende Glyceridgemisch erhalten wurde durch Veresterung von Glycerin mit Carbonsäuren, Umesterung von Triglyceriden mit Glycerin, enzymatische Spaltung von Triglyceriden oder Fraktionierung der resultierenden Gemische und vorzugsweise die folgende Zusammensetzung, jeweils bezogen auf die Gesamtmenge an Glyceriden und freien Säuren, aufweist:
- 20 bis 99, insbesondere 40 bis 70 Gew.-% Monoglyceride;
- 0,4 bis 95, insbesondere 25 bis 45 Gew.-% Diglyceride;
- 0,3 bis 20, insbesondere 3 bis 10 Gew.-% Triglyceride;
- 0,3 bis 5, insbesondere 0,5 bis 3 Gew.-% freie Fettsäuren.

4. Verfahren nach irgendeinem der Ansprüche 1 bis 3, dadurch gekennzeichnet, daß sich die Glyceride bzw. Glyceridgemische von gegebenenfalls ungesättigten aliphatischen Carbonsäuren (Fettsäuren) mit 4 bis 22, vorzugsweise 6 bis 18, Kohlenstoffatomen ableiten.

5. Verfahren nach irgendeinem der Ansprüche 1 bis 4, dadurch gekennzeichnet, daß bei Lösungsmittel (a) gemäß Anspruch 1 die Verbindungen des Typs A Ester, Lactone und Ketone, vorzugsweise mit einer Wasserlöslichkeit von höchstens 10 Gew.-%, sind und die Verbindungen des Typs B und/oder C in einer Gesamtmenge von 0 bis 10, vorzugsweise 0 bis 5 Gew.-% vorliegen; in Lösungsmittel (b) 40 bis 60 Gew.% Verbindungen des Typs D und/oder E, vorzugsweise nur Verbindungen des Typs D, vorliegen; im Falle von Lösungsmittel (c) Verbindungen des Typs E alleine oder in Kombination mit bis zu 60 Gew.-% an Verbindungen des Typs D vorliegen; und im Falle von Lösungsmittel (d) Propan und/oder Butan in Mengen von 30 bis 70 Gew.-% anwesend sind und vorzugsweise bei einem Druck von 2 bis 70 bar gearbeitet wird.

6. Verfahren nach irgendeinem der Ansprüche 1 bis 5, dadurch gekennzeichnet, daß die Verbindungen des Typs A, B und C einen Siedepunkt von höchstens 160°C, vorzugsweise höchstens 130°C, aufweisen.

7. Verfahren nach irgendeinem der Ansprüche 1 bis 6, dadurch gekennzeichnet, daß die Verbindungen des Typs A ausgewählt werden aus aliphatischen Estern und Lactonen mit insgesamt 4 bis 8 Kohlenstoffatomen, insbesondere aus Ethylacetat, Isopropylacetat und Butylacetat; und/oder die Verbindungen des Typs B ausgewählt werden aus (cyclo)aliphatischen Ketonen mit insgesamt 3 bis 6 Kohlenstoffatomen, insbesondere aus Aceton, Butanon und 3-Pentanon; und/oder die Verbindungen des Typs C ausgewählt werden aus ein- und zweiwertigen (cyclo)aliphatischen Alkoholen mit 1 bis 6 Kohlenstoffatomen, insbesondere aus Methanol, Ethanol, Isopropanol, n-Butanol und Ethylenglykol; und/oder die Verbindungen des Typs D ausgewählt werden aus (cyclo)aliphatischen Kohlenwasserstoffen mit 2 bis 12 Kohlenstoffatomen, vorzugsweise 3 bis 7 Kohlenstoffatomen und Mischungen derselben, und insbesondere aus Propan, Butan und Isohexan; und/ oder die Verbindungen des Typs E ausgewählt werden aus (cyclo)aliphatischen Ethern mit 3 bis 10, vorzugsweise 3 bis 6 Kohlenstoffatomen und 1 bis 3 Ethergruppen, insbesondere aus Diethylether, Di(iso)propylether, Tetrahydrofuran und Dioxan.

8. Verfahren nach irgendeinem der Ansprüche 1 bis 7, dadurch gekennzeichnet, daß das Verhältnis von Wasser zu Glyceridlösung so eingestellt wird, daß die Wassermenge das ein- bis achtfache, vorzugsweise das zwei- bis fünffache der Menge beträgt, die zur Erzeugung von Zweiphasigkeit in der letzten Extraktionsstufe erforderlich ist.

9. Verfahren nach irgendeinem der Ansprüche 1 bis 8, dadurch gekennzeichnet, daß das zur Extraktion verwendete Wasser bereits die Gleichgewichtskonzentration ±2% an den Verbindungen des Typs B und/oder C enthält, die sich im Verlauf der Extraktion in der wäßrigen Phase einstellen würde.

10. Verfahren nach irgendeinem der Ansprüche 1 bis 9, dadurch gekennzeichnet, daß es kontinuierlich, vorzugsweise im Gegenstrom durchgeführt wird.

11. Verfahren nach irgendeinem der Ansprüche 1 bis 10, dadurch gekennzeichnet, daß es bei Temperaturen von 20°C bis 160°C durchgeführt wird.

12. Verfahren nach irgendeinem der Ansprüche 1 bis 11, dadurch gekennzeichnet, daß die Extraktion in einer gepackten Säule im Gegenstrom, in einer Kolonne mit rotierenden Einbauten, in einer Mischer-Abscheider-Batterie, in einem Zentrifugalextraktor oder in einer Siebbodenkolonne durchgeführt wird.
